(19) **Europäisches Patentamt European Patent Office Office européen des brevets**

(11) **EP 4 259 081 B1**

(12) **FASCICULE DE BREVET EUROPEEN**

(45) Date de publication et mention de la délivrance du brevet:
**25.02.2026 Bulletin 2026/09**

(21) Numéro de dépôt: **21824593.4**

(22) Date de dépôt: **13.12.2021**

(51) Classification Internationale des Brevets (IPC):
**A61K 8/33** *(2006.01)*        **A61K 8/362** *(2006.01)*
**A61K 8/49** *(2006.01)*        **A61K 8/9794** *(2017.01)*
**A61K 36/88** *(2006.01)*       **A61P 17/18** *(2006.01)*

(52) Classification Coopérative des Brevets (CPC):
**A61K 8/498; A61K 8/33; A61K 8/362;
A61K 8/9794; A61K 36/88; A61P 17/18**

(86) Numéro de dépôt international:
**PCT/EP2021/085487**

(87) Numéro de publication internationale:
**WO 2022/128915 (23.06.2022 Gazette 2022/25)**

(54) **EXTRAIT D'AU MOINS UNE PLANTE DE L'ESPÈCE CROCUS SATIVUS COMPRENANT UN FORT TAUX DE CROCINES ET UN FAIBLE TAUX DE SAFRANAL, ET SON UTILISATION COSMÉTIQUE À TITRE D'AGENT ANTIOXYDANT**

EXTRAKT AUS MINDESTENS EINER PFLANZE DER ART CROCUS SATIVUS, DIE EINEN HOHEN GEHALT AN CROCINEN UND EINEN NIEDRIGEN GEHALT AN SAFRANAL ENTHÄLT, UND SEINE VERWENDUNG ALS ANTIOXIDANS

EXTRACT OF AT LEAST ONE PLANT OF THE SPECIES CROCUS SATIVUS CONTAINING A HIGH LEVEL OF CROCINES AND A LOW LEVEL OF SAFRANAL, AND ITS USE AS AN ANTIOXIDANT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.12.2020 FR 2013205**

(43) Date de publication de la demande:
**18.10.2023 Bulletin 2023/42**

(73) Titulaire: **L'OREAL**
**75008 Paris (FR)**

(72) Inventeurs:
• **RONDOT, Christophe**
 **93601 AULNAY-SOUS-BOIS (FR)**
• **NGOM, Saliou**
 **93601 AULNAY-SOUS-BOIS (FR)**

(74) Mandataire: **L'Oreal**
**Service D.I.P.I.**
**9, rue Pierre Dreyfus**
**92110 Clichy (FR)**

(56) Documents cités:
**EP-A1- 3 446 678        WO-A1-2018/020013
FR-A1- 2 949 975        FR-A1- 3 001 891
JP-A- 2005 041 811      JP-A- 2005 343 882**

• **LAGE M ET AL: "Quantification of saffron (Crocus sativus L.) metabolites crocins, picrocrocin and safranal for quality determination of the spice grown under different environmental Moroccan conditions", SCIENTIA HORTICULTURAE, ELSEVIER, AMSTERDAM, NL, vol. 121, no. 3, 2 July 2009 (2009-07-02), pages 366 - 373, XP026096709, ISSN: 0304-4238, [retrieved on 20090418], DOI: 10.1016/J.SCIENTA.2009.02.017**
• **CROZET A ET AL: "L. (Iridaceae), le safran (I)", PHYTOTHÉRAPIE ; DE LA RECHERCHE À LA PRATIQUE, SPRINGER-VERLAG, PA, vol. 10, no. 2, 29 March 2012 (2012-03-29), pages 121 - 125, XP035051605, ISSN: 1765-2847, DOI: 10.1007/S10298-012-0696-Z**

- URBANI ELEONORA ET AL: "Investigation on secondary metabolite content and antioxidant activity of commercial saffron powder", EUROPEAN FOOD RESEARCH AND TECHNOLOGY, SPRINGER BERLIN HEIDELBERG, BERLIN/HEIDELBERG, vol. 242, no. 6, 15 April 2016 (2016-04-15), pages 987 - 993, XP035868126, ISSN: 1438-2377, [retrieved on 20160415], DOI: 10.1007/S00217-016-2687-Z

**Description**

La présente invention concerne le domaine du soin de la peau.

Plus particulièrement, la présente invention vise à proposer un nouvel actif utilisé en tant qu'agent antioxydant.

L'utilisation d'antioxydants dans la cosmétique est très importante et de plus en plus généralisée. Ainsi, les antioxydants permettent de combattre les radicaux libres ($O_2$°-, OH°, $^1O_2$...) induisant notamment un vieillissement cellulaire.

Ils peuvent ainsi être utilisés dans différents axes cosmétiques tels que l'anti-âge, la protection contre les stress oxydatifs et notamment exogènes dus à l'exposition au soleil, à l'environnement (la pollution, la fumée), la prévention de la pigmentation (la synthèse de mélanine est un processus oxydatif.

Cette activité antioxydante est en particulier avantageuse pour limiter la peroxydation des lipides de surfaces de la peau et en particulier la peroxydation photoinduite des lipides d'origine sébacée, comme le squalène.

On sait en effet que les lipides se trouvant à la surface de la peau sont soumis en permanence à des agressions extérieures et notamment l'air, les polluants atmosphériques et les rayonnements visibles et surtout ultraviolets (UV) et que les plus exposés aux agressions extérieures sont ceux contenus dans les sécrétions grasses de la peau comme le sébum, riche en squalène. La présence dans les molécules de squalène de six doubles liaisons rend ces molécules sensibles aux phénomènes d'oxydation. Ainsi, lors d'une exposition prolongée aux UV, le squalène se photoperoxyde pour donner des peroxydes de squalène.

Cette production élevée de peroxydes de squalène, provoque notamment une série de dégradations en chaîne en particulier sur la peau, donnant naissance à de nombreux désordres cutanés.

Ainsi ces peroxydes de squalène interviennent notamment dans le vieillissement prématuré de la peau comme décrit par Keiko OH Sawa et al. (J. Toxicol. Sc., 1984 : 19, pp 151-159).

De nombreux antioxydants existent déjà tels que le tocophérol (vitamine E) ou ses dérivés, la vitamine C ou ses dérivés, les caroténoïdes, l'ubiquinone, le thé vert, etc. Fr 3 001 891 A1, FR 2 949 975 A1, JP 2005 041811 A et JP 2005 343882 A décrivent des extraits issus de *Crocus salivus L.* permettant de lutter contre le vieillissement de la peau, du en particulier au stress oxydatif, aux rayons UV ou a la pollution. Cependant, ils ne donnent pas les concentrations en crocine(s) et safrane dans les extraits utilisés.

Toutefois, il existe un besoin permanent de disposer de nouveaux actifs susceptibles d'exercer une action cosmétique antioxydante et notamment de lutter contre le stress oxydatif de la peau.

De manière surprenante les inventeurs ont démontré qu'un extrait d'au moins une plante de l'espèce *Crocus sativus* comprenant (i) au moins 20% en poids sec d'une ou plusieurs crocine(s) par rapport au poids total des composants de l'extrait sec et (ii) moins de 0,08% en poids sec de safranal par rapport au poids total des composants de l'extrait sec, permet de lutter contre la péroxydation du squalène, et présente donc une activité antioxydante tout en ayant une odeur agréable et de faible intensité, et donc compatible avec une utilisation au sein de produits cosmétiques.

Il est connu de l'art antérieur CN108542840A un extrait de safran (*Crocus sativus*) comprenant 10% en poids de crocines ayant des propriétés antioxydantes, cependant cet extrait présente une activité antioxydante bien plus faible, notamment sur sa capacité à lutter contre la péroxydation du squalène comparativement à l'extrait selon l'invention comme le montre l'exemple 4 de la présente demande.

En outre il est connu de la littérature (Quality and functionality of saffron quality control, species assortment and affinity of extract and isolated saffron compounds to NMDA and sigma-1 receptors, Matthias Lechtenberg, Dirk Schepmann, Michael Niehues, Nils Hellenbrand, Bernhard Wünsch, Andreas Hensel, publié en juin 2008 dans le journal Planta Medica), un extrait de safran (*Crocus sativus*) comprenant 27% en poids de crocines, et 0,098% en poids de safranal. Cependant, cet extrait n'est pas divulgué comme présentant des propriétés antioxydantes. En outre, sa teneur en safranal supérieure à 0,08% génère une odeur puissante et désagréable ne permettant pas une utilisation au sein de produits cosmétiques comme le montre l'exemple 5 de la présente demande.

Ainsi, la présente invention a pour premier objet un extrait d'au moins une plante de l'espèce *Crocus sativus* comprenant (i) au moins 20% en poids sec d'une ou plusieurs crocine(s) par rapport au poids total des composants de l'extrait sec et (ii) moins de 0,08% en poids sec de safranal par rapport au poids total des composants de l'extrait sec.

La présente invention a également pour objet une composition comprenant, dans un milieu physiologiquement acceptable ledit extrait.

Un autre des objets de la présente invention concerne un procédé de traitement cosmétique comprenant l'application dudit extrait ou de ladite composition sur les matières kératiniques, notamment la peau pour le soin de la peau, et notamment :

- pour prévenir et/ou traiter les désordres cutanés induits par le stress oxydatif, notamment induit par les rayons UV et/ou la pollution en particulier atmosphérique ;
- et/ou pour prévenir et/ou traiter les signes du vieillissement cutané ;
- et/ou pour prévenir et/ou traiter l'aspect terne et/ou hétérogène du teint ;

- et/ou pour améliorer l'éclat et/ou l'uniformité du teint ;
- et/ou pour prévenir les désordres pigmentaires tels que le lentigo actinique ou les hyperpigmentations post-inflammatoires.

La présente invention se rapporte en outre à l'utilisation cosmétique dudit extrait pour le soin de la peau, et notamment,

- pour prévenir et/ou traiter les désordres cutanés induits par le stress oxydatif, notamment induit par les rayons UV et/ou la pollution en particulier atmosphérique;
- et/ou pour prévenir et/ou traiter les signes du vieillissement cutané
- et/ou pour prévenir et/ou traiter l'aspect terne et/ou hétérogène du teint ;
- et/ou pour améliorer l'éclat et/ou l'uniformité du teint ;
- et/ou pour prévenir les désordres pigmentaires tels que le lentigo actinique ou les hyperpigmentations post-inflammatoires.

La présente invention concerne également l'utilisation cosmétique dudit extrait comme agent antioxydant.

Un autre objet de la présente invention est un procédé de préparation dudit extrait d'au moins une plante de l'espèce *Crocus sativus* selon l'invention comprenant au moins une étape d'extraction d'une partie d'au moins une plante de l'espèce *Crocus sativus à* l'aide d'un solvant aqueux choisi parmi l'eau ou un mélange d'eau et d'un ou plusieurs solvant(s) organique(s) miscible(s) dans l'eau, de préférence à l'aide d'un mélange éthanol/eau dans un ratio en volume de 1/1.

**Définition**

Par « matière kératinique », on entend la peau humaine.

Par « la peau », on entend l'ensemble de la peau du corps, et le cuir chevelu et de manière préférée, la peau du visage, du décolleté, du cou, des bras et avant-bras, voire de manière plus préférée encore, la peau du visage (en particulier du front, nez, joues, menton), du décolleté et du cou.

Par « prévenir » ou « prévention » on entend selon l'invention le fait de réduire le risque de survenue ou de ralentir la survenue d'un phénomène donné.

Par « traiter » ou « traitement » on entend toute action visant à améliorer le confort, le bien-être d'un individu, ce terme couvre donc aussi bien atténuer, soulager que curer.

Le terme « antioxydant » désigne un composé qui diminue ou empêche l'oxydation d'autres composés chimiques.

L'oxydation fait partie d'une réaction redox qui transfère des électrons d'une substance à un agent oxydant. Cette réaction peut produire des radicaux libres. Un agent antioxydant peut notamment être un agent antiradicalaire.

On entend par « radicaux libres » au sens de la présente invention des espèces chimiques avec un ou plusieurs électrons non appariés sur sa couche externe comme par exemple les radicaux libres de l'oxygène.

Les radicaux libres de l'oxygène sont notamment :

- l'oxygène singulet • O-O •;
- l'anion radical superoxyde $O_2$ • -;
- le radical hydroxyle HO •;
- le radical hydroperoxyle $HO_2$ •;
- le radical peroxyde (ROO •) et le radical alcoxy (RO •) où R est une chaîne carbonée.

On entend par « antiradicalaire » au sens de la présente invention un composé qui neutralise les radicaux libres formés.

Les termes « stress oxydatif » au sens de la présente invention couvrent tous les dommages provoqués par une augmentation des radicaux libres de l'oxygène chez un sujet.

**Description détaillée**

*Extrait de Crocus sativus (safran)*

La présente invention concerne un extrait d'au moins une plante de l'espèce *Crocus sativus* comprenant (i) au moins 20% en poids sec d'une ou plusieurs crocine(s) par rapport au poids total des composants de l'extrait sec et (ii) moins de 0,08% en poids sec de safranal par rapport au poids total des composants de l'extrait sec.

Le *Crocus sativus* (*Crocus sativus* L., 1753) ou crocus cultivé, crocus à safran, Safran, Safran cultivé, est une plante géophyte de la famille des Iridaceae, dont on extrait le safran. *Crocus sativus* appartient à un groupe d'une dizaine d'espèces de crocus méditerranéens à partir desquels on produisait le safran dans l'antiquité.

*Crocus sativus* est une plante vivace géophyte de 10 à 30 cm, glabrescente. Son corme (tige souterraine de réserve ressemblant à un bulbe) est très volumineux pour un crocus (de moins de 1 cm de circonférence pour les caïeux à près de 20 cm) et subglobuleux. Les tuniques ou tissus fibreux qui entourent et protègent le corme sont composées de fibres réticulées en mailles longues et étroites. Les feuilles linéaires apparaissent en automne, avant, avec, ou après les fleurs, et poussent continuellement jusqu'à l'entrée en repos végétatif à la fin du printemps. Elles peuvent alors mesurer une soixantaine de cm pour quelques mm de largeur. Au nombre de 1 à 10 environ par bourgeon, elles forment des touffes, d'abord dressées, puis retombantes, d'un beau vert franc. Chaque corme produit éventuellement jusqu'à une dizaine de fleurs réunies en inflorescence. Tiges et spathes restent souterraines et sont donc invisibles. Les fleurs, organisées sur une symétrie ternaire, sont spectaculaires, grandes, lilas, parfumées. Leur périanthe est composé de 6 tépales (3 pétales et 3 sépales semblables) fusionnés en une gorge violacée pour former le tube du périanthe (pris à tort pour la "tige" de la fleur). Les anthères sont jaune vif, au nombre de 3, de moitié plus longues que leur filet. Le pistil qui prolonge l'ovaire souterrain, se subdivise en 3 parties terminales, les stigmates. Les stigmates du *Crocus sativus* sont hypertrophiés, écarlates, odorants, évasés en trompette crénelée. Ils retombent souvent hors du périanthe sous l'effet de leur taille. Ils sont utilisés pour produire une épice rare et précieuse : le safran. Dans l'hémisphère nord, la floraison se produit entre septembre et novembre et dure de 2 à 6 semaines. Le *Crocus sativus* étant triploïde, il ne produit pas de graines et est donc exclusivement multiplié par voie végétative.

Le safran, en particulier les stigmates, sont connus pour leurs usages alimentaires comme condiment.

Le safran est notamment originaire d'Italie et de l'Orient notamment le Maroc, mais il peut être cultivé également en dans les régions de Grèce et de Crète, de Suisse, d'Iran.

Au Maroc, les principaux sites de production du safran correspondent à 2 terroirs très localisés du massif Siroua massif volcanique situé à la jointure de l'Anti-Atlas et du Haut-Atlas, le terroir de Taliouine, le terroir de Tazenakht, caractérisés par un fort isolement géographique et par la forte dispersion des bassins de culture, concentrés autour des points d'eau. Parmi les terroirs de production secondaire, on peut citer le terroir de l'Ourika.

Avantageusement, le *Crocus sativus* utilisé dans le cadre de la présente invention provient du Maroc comme celui provenant de la vallée de l'Ourika, en particulier située à 35 km au Sud-Ouest de Marrakech, près d'Oukaimeden. La vallée de l'Ourika se trouve au niveau du versant Nord-Ouest du Haut-Atlas. La récolte se fait en période automnale notamment du 15 octobre au 15 novembre. A titre d'exemple de safran (*Crocus sativus*) provenant de la vallée de l'Ourika, on peut citer notamment ceux provenant des Jardins Yves Saint Laurent, ou encore ceux de la Safranière de l'Ourika.

De préférence, ladite ou lesdites crocine(s) (i) est(sont) présente(s) entre 20% et 40% en poids sec par rapport au poids total des composants de l'extrait sec, encore plus préférentiellement, entre 25% et 35% en poids sec par rapport au poids total des composants de l'extrait sec.

De préférence, ledit safranal (ii) est présent à moins de 0,07% en poids sec par rapport au poids total des composants de l'extrait sec, encore plus préférentiellement moins de 0,06% en poids sec par rapport au poids total des composants de l'extrait sec. Dans un mode de réalisation préféré, ledit extrait est totalement exempt de safranal i.e. 0%.

La ou les crocine(s) présente(s) dans ledit extrait est(sont) choisie(s) parmi :

- la bis-gentiobiosyl-E-crocétine ;
- la gentiobiosyl-glucosyl-E-crocétine ;
- la bis-glucosyl-E-crocétine ;
- la bis-gentiobiosyl-13-Z-crocétine ;
- la gentiobiosyl-glucosyl-13-Z-crocétine ;
- et leurs mélanges.

La ou les crocine(s) est(sont) sous la forme d'un mélange comprenant :

- entre 10% et 30% en poids sec de bis-gentiobiosyl-E-crocétine ;
- entre 5% et 10% en poids sec de gentiobiosyl-glucosyl-E-crocétine ;
- entre 0,1% et 5% en poids sec de bis-glucosyl-E-crocétine ;
- entre 0,05% et 2% en poids sec de bis-gentiobiosyl-13-Z-crocétine ;

par rapport au poids total des composants de l'extrait sec.

De préférence, la ou les crocine(s) est(sont) sous la forme d'un mélange comprenant :

- entre 60% et 70% en poids sec de bis-gentiobiosyl-E-crocétine ;
- entre 20% et 30% en poids sec de gentiobiosyl-glucosyl-E-crocétine ;

- entre 5% et 10% en poids sec de bis-glucosyl-E-crocétine ;
- entre 2% et 6% en poids sec de bis-gentiobiosyl-13-Z-crocétine ;

par rapport au poids total des crocines de l'extrait sec.

Ledit extrait peut comprendre en outre de la picrocrocine. La picrocrocine peut être présente dans ledit extrait en une concentration comprise entre 10% et 20% en poids par rapport au poids total des composants de l'extrait sec.

Avantageusement, ledit extrait est obtenu par extraction d'une partie d'au moins une plante de l'espèce *Crocus sativus* à l'aide d'un solvant aqueux. L'étape d'extraction peut être répétée au moins une seconde fois, notamment l'étape d'extraction peut être réalisée deux fois.

Par « partie de plante de l'espèce *Crocus sativus* » on entend les fleurs, les tiges, les feuilles, les stigmates, les pistils, de préférence la(les) partie(s) de plante(s) de l'espèce Crocus sativus à partir de laquelle l'extraction est réalisée est(sont) le(s) pistil(s).

Par « solvant aqueux » on entend de l'eau ou un mélange d'eau et d'un ou plusieurs solvant(s) organique(s) miscible(s) dans l'eau. Les solvants organiques miscibles dans l'eau peuvent être choisis parmi les monoalcools, linéaires ou ramifiés, en C1-C6 tels que l'éthanol, l'isopropanol, le tertio-butanol; les polyols tels que le glycérol, le propylèneglycol, l'hexylène glycol (ou 2-méthyl-2,4-pentanediol), et les polyéthylèneglycols; les éthers de polyols comme le monométhyléther de dipropylèneglycol; et leurs mélanges.

De manière encore plus préférée, ledit extrait est obtenu par extraction à l'aide d'un mélange éthanol/eau dans un ratio en volume de 1/1.

Avantageusement, l'extraction est réalisée pendant environ une durée variant de 30 mn à 8 heures, de préférence entre 1 et 6 heures, plus particulièrement entre 3 et 5 heures tel que 4 heures, notamment sous agitation par exemple à l'aide d'un agitateur à pâle ou magnétique.

L'extraction est en particulier une macération à température ambiante, par exemple à 25°C.

Ledit extrait est notamment obtenu par extraction de 10g de pistils de *Crocus sativus* par litre de solvant à 1000g de pistils de *Crocus sativus* par litre de solvant, de préférence de 20g de pistils de *Crocus sativus* par litre de solvant à 500g de pistils de *Crocus sativus* par litre de solvant, encore mieux de 50g de pistils de *Crocus sativus* par litre de solvant à 200g de pistils de *Crocus sativus* par litre de solvant, tel que 100g de pistils de *Crocus sativus* par litre de solvant.

L'extrait ainsi obtenu peut être séché et se présenter alors sous la forme d'un extrait sec, après élimination d'un(des) solvant(s), par exemple par évaporation d'un ou plusieurs solvants organiques miscibles dans l'eau notamment à l'aide d'un évaporateur rotatif et/ou par évaporation de l'eau notamment à l'aide d'un évaporateur rotatif, par lyophilisation, ou par atomisation, ou une combinaison de ces méthodes.

On entend par « extrait sec » au sens de la présente invention, un extrait comprenant moins de 10% en poids d'eau, de préférence moins de 7% en poids d'eau, encore mieux moins de 5%, encore plus préférentiellement moins de 2% en poids d'eau, voire totalement exempte d'eau (0%).

Dans un mode de réalisation préféré, l'extrait obtenu à l'issue de l'étape d'extraction peut subir :

i) une étape de filtration, en particulier sur une toile de nylon présentant une taille de pores comprise entre $50 \mu m$ et $200 \mu m$, et/ou

ii) une étape d'empâtage dans de la cellulose, et/ou

iii) une ou plusieurs étapes de clarification, de préférence l'extrait obtenu à l'issue de l'étape d'extraction peut subir une première étape de clarification sur un lit de cellulose et toile de coton présentant une taille de pores comprise entre $5 \mu m$ et $15 \mu m$ ainsi qu'une seconde étape de clarification sur une plaque de cellulose présentant une taille de pores comprise entre $0,1 jum$ et $3 \mu m$, de préférence entre $0,8 \mu m$ et $0,5 \mu m$ ; et/ou

iv) une étape de stérilisation, notamment par passage à travers une cartouche de $0,2 \mu m$.

Dans un mode de réalisation préféré, chacune des étapes précitées (extraction telle que par macération, filtration empâtage, clarification, évaporation, etc...) sont réalisées à l'abri de la lumière.

Préalablement à l'étape d'extraction, les pistils de *Crocus sativus* peuvent être séchés et/ou broyés par exemple sous la forme de particules de taille inférieure ou égale à 5mm, de préférence de taille inférieure ou égale à 2 mm.

La présente invention concerne également un procédé de préparation dudit extrait d'au moins une plante de l'espèce *Crocus sativus* selon l'invention comprenant au moins une étape d'extraction d'une partie d'au moins une plante de l'espèce *Crocus sativus* à l'aide d'un solvant aqueux choisi parmi l'eau ou un mélange d'eau et d'un ou plusieurs solvant(s) organique(s) miscible(s) dans l'eau, de préférence à l'aide d'un mélange éthanol/eau dans un ratio en volume de 1/1.

Dans ce procédé, l'extrait obtenu à l'issue de cette étape d'extraction peut subir les étapes suivantes, de préférence consécutives :

i) une étape de filtration, en particulier sur une toile de nylon présentant une taille de pores comprise entre $50 \mu m$ et

200µm, et/ou

ii) une étape d'empâtage dans de la cellulose, et/ou

iii) une ou plusieurs étapes de clarification, de préférence l'extrait obtenu à l'issue de l'étape d'extraction peut subir une première étape de clarification sur un lit de cellulose et toile de coton présentant une taille de pores comprise entre 5µm et 15µm ainsi qu'une seconde étape de clarification sur une plaque de cellulose présentant une taille de pores comprise entre 0,1µm et 3µm, de préférence entre 0,8µm et 0,5µm ; et/ou

iv) une étape de stérilisation, notamment par passage à travers une cartouche de 0,2µm.

A l'issue de ladite étape d'extraction et éventuellement des étapes i) et/ou ii) et/ou iii) et/ou iv), de préférence consécutives, ledit extrait peut subir une étape de séchage qui comprend l'élimination d'un(des) solvant(s), par exemple par évaporation d'un ou plusieurs solvants organiques miscibles dans l'eau notamment à l'aide d'un évaporateur rotatif et/ou par évaporation de l'eau notamment à l'aide d'un évaporateur rotatif, par lyophilisation, ou par atomisation, ou une combinaison de ces méthodes.

*Composition*

La présente invention concerne également une composition comprenant, dans un milieu physiologiquement acceptable l'extrait d'au moins une plante de l'espèce *Crocus sativus* selon l'invention.

Ledit extrait peut être présent dans la composition en une concentration comprise entre 0,0001% et 20% en poids d'extrait sec, de préférence entre 0,001% et 10% en poids d'extrait sec, encore mieux entre 0,001% et 5% en poids d'extrait sec par rapport au poids total de la composition.

Par « milieu physiologiquement acceptable », on comprend un milieu compatible avec les matières kératiniques, et en particulier la peau.

Plus particulièrement, ledit milieu physiologiquement acceptable peut comprendre de l'eau et/ou un ou plusieurs solvants organiques miscibles à l'eau qui peuvent être choisis parmi les monoalcools, linéaires ou ramifiés, en C1-C6 tels que l'éthanol, l'isopropanol, le tertio-butanol; les polyols tels que le glycérol, le propylèneglycol, l'hexylène glycol (ou 2-méthyl-2,4-pentanediol), et les polyéthylèneglycols; les éthers de polyols comme le monométhyléther de dipropylèneglycol; et leurs mélanges.

Préférentiellement, la composition selon l'invention présente une teneur en eau allant de 20% à 95% en poids, encore mieux de 30% à 70% en poids par rapport au poids total de la composition.

Avantageusement, la composition comprend un ou plusieurs solvants organiques miscibles à l'eau en une teneur allant de 0.5% à 25% en poids, de préférence, de 5% à 20% en poids, encore mieux de 10% à 15% en poids par rapport au poids total de la composition.

La composition selon l'invention peut comprendre en outre tous les adjuvants usuellement employés dans le domaine d'application envisagée.

On peut notamment citer; les solvants organiques, notamment les alcools en C1-C6 et les esters d'acide carboxylique en C2-C10; les huiles carbonées et/ou siliconées, d'origine minérale, animale et/ou végétale; les cires, les pigments, les charges, les colorants, les tensioactifs, les émulsionnants, les co-émulsionnants; les actifs cosmétiques distinct de l'extrait selon l'invention, les filtres UV, les polymères, les gélifiants hydrophiles ou lipophiles, les épaississants, les conservateurs, les parfums, les bactéricides, les absorbeurs d'odeur, les antioxydants.

Ces éventuels adjuvants peuvent être présents dans la composition à raison de 0,001% à 80% en poids, de préférence 0,01% à 30% en poids, encore mieux de 0,1% à 20 % par rapport au poids total de la composition. Ces adjuvants, selon leur nature, peuvent être introduits dans la phase grasse, dans la phase aqueuse et/ou dans les vésicules lipidiques.

Bien entendu, l'homme du métier veillera à choisir ce ou ces éventuels ingrédients et/ou actifs complémentaires, et/ou leur quantité, de manière telle que les propriétés avantageuses de l'extrait selon l'invention, ne soient pas, ou substantiellement pas, altérées par l'adjonction envisagée.

Les compositions selon l'invention peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules, ces sphérules pouvant être des vésicules lipidiques de type ionique et/ou non ionique (liposomes, niosomes, oléosomes). Ces compositions sont préparées selon les méthodes usuelles.

Avantageusement les compositions selon l'invention se présentent sous forme de gel, ou d'émulsion, de poudre ou de pâte. En outre, la composition selon l'invention peut être plus ou moins fluide et avoir l'aspect d'une crème blanche ou colorée, d'une pommade, d'un lait, d'une lotion, d'un sérum, d'une pâte, d'un gel moussant, d'un gommage, d'un masque, d'un soin, d'un tonique ou d'une mousse. Elle peut être éventuellement appliquée sur la peau sous forme d'aérosol. Elle peut aussi se présenter sous forme solide, et par exemple sous forme de stick.

Une composition selon l'invention peut comprendre une phase huileuse.

Comme huiles utilisables dans la composition de l'invention, on peut citer par exemple :

- les huiles hydrocarbonées,
- les esters et les éthers de synthèse, notamment d'acides gras, comme les huiles de formules R'COOR2 et R'COR2 dans laquelle R' représente le reste d'un acide gras comportant de 8 à 29 atomes de carbone, et R2 représente une chaîne hydrocarbonée, ramifiée ou non, contenant de 3 à 30 atomes de carbone ;
- les hydrocarbures linéaires ou ramifiés, d'origine minérale ou synthétique ;
- les alcools gras ayant de 8 à 26 atomes de carbone ;
- les huiles fluorées partiellement hydrocarbonées et/ou siliconées ;
- les huiles de silicone ;
- leurs mélanges.

On entend par huile hydrocarbonée dans la liste des huiles citées ci-dessus, toute huile comportant majoritairement des atomes de carbone et d'hydrogène.

La phase huileuse peut renfermer d' autres corps gras pouvant être présents dans la phase huileuse sont par exemple les acides gras comportant de 8 à 30 atomes de carbone ; les cires ; les résines de silicone ; et les élastomères de silicone. Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 60 % en poids par rapport au poids total de la composition. Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co- émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols, et les alkyl-dimethicone copolyols. On peut aussi utiliser comme tensioactif d'émulsions E/H, un organopolysiloxane solide élastomère réticulé comportant au moins un groupement oxyalkyléné.

Pour les émulsions H/E, on peut citer par exemple comme émulsionnants, les émulsionnants non ioniques.

Les compositions selon l'invention pourront être appliquées directement sur la peau ou, de façon alternative, sur des supports cosmétiques de type occlusif ou non occlusif, destinés à être appliqués de façon localisée sur la peau. A titre d'exemples de supports cosmétiques, non limitatifs, on peut notamment citer un patch, une lingette, un roll-on et un stylo. La composition peut être rincée ou non après avoir été appliquée sur la peau.

*Utilisations et procédé de traitement cosmétique*

Un autre des objets de la présente invention concerne un procédé de traitement cosmétique comprenant l'application dudit extrait ou de ladite composition sur les matières kératiniques, notamment la peau pour le soin de la peau, et notamment pour:

- pour prévenir et/ou traiter les désordres cutanés induits par le stress oxydatif, notamment induit par la pollution en particulier atmosphérique ;
- et/ou pour prévenir et/ou traiter les signes du vieillissement cutané ;
- et/ou pour prévenir et/ou traiter l'aspect terne et/ou hétérogène du teint ;
- pour améliorer l'éclat et/ou l'uniformité du teint ;
- pour prévenir les désordres pigmentaires tels que le lentigo actinique

La présente invention se rapporte en outre à l'utilisation cosmétique dudit extrait pour le soin de la peau, et notamment :

- pour prévenir et/ou traiter les désordres cutanés induits par le stress oxydatif, notamment induit par la pollution en particulier atmosphérique;
- et/ou pour prévenir et/ou traiter les signes du vieillissement cutané
- et/ou pour prévenir et/ou traiter l'aspect terne et/ou hétérogène du teint ;
- pour améliorer l'éclat et/ou l'uniformité du teint ;
- pour prévenir les désordres pigmentaires tels que le lentigo actinique

La présente invention concerne également l'utilisation cosmétique dudit extrait comme agent antioxydant.

Parmi les désordres cutanés désordres cutanés induits par le stress oxydatif, notamment induit par la pollution en particulier atmosphérique on peut citer notamment les signes du vieillissement cutané.

Parmi les signes du vieillissement cutané on peut citer notamment un amincissement de la peau, une perte de fermeté, une perte d'élasticité, une perte de densité, ou une perte de tonicité de la peau, une altération de l'aspect de surface de la peau, une apparition d'un microrelief marqué de la peau, une apparition de rugosité, une formation et/ou une présence de ridules et/ou de rides, une altération de l'éclat du teint de la peau, un aspect papyracé de la peau, un affaissement de la peau, ou un flétrissement de la peau.

Dans toute la description, y compris les revendications, l'expression « comprenant un » doit être comprise comme étant synonyme de « comprenant au moins un », sauf si le contraire est spécifié.

En outre l'expression « au moins un » doit être comprise comme étant synonyme de « un ou plusieurs » sauf si le contraire est spécifié.

Les expressions « plus de », « compris entre ... et ... » et « allant de ... à ... » doivent se comprendre bornes incluses, sauf si le contraire est spécifié.

Les exemples et figures qui suivent sont présentés à titre illustratif et non limitatif de l'invention. Les composés sont, selon le cas, cités en noms chimiques ou en noms CTFA (International Cosmetic Ingredient Dictionary and Handbook).

## Exemples

### Exemple 1 - Préparation d'un extrait de pistils de *Crocus sativus* provenant de la vallée de l'Ourika comprenant 29% de crocines et 0% de safranal (selon l'invention).

Le Safran sous forme de pistils entiers séchés de fleurs de *Crocus Sativus* provenant de la vallée de l'Ourika, récolté en 2019, est broyé avec un broyeur à couteaux sur une grille de 1 mm. 1,9 kg de la poudre orangée ainsi obtenue est extraite sous agitation à l'abri de la lumière avec 19 litre d'un mélange éthanol-eau (1/1) à 25°C pendant 4h. Le mélange est préfiltré sur une toile Nylon de 195 $\mu$m dans un filtre clos. La biomasse épuisée est dépotée. Le pré-filtrat, réempaté dans de la cellulose (Vitacel LC200) comme adjuvant (alluvionnage), est pré-clarifié sur un lit de cellulose dans un filtre clos équipé d'une toile coton 10$\mu$m, puis est clarifié en série dans un filtre cloche équipé d'une plaque en cellulose de 0.8-0.5$\mu$m avant finalement d'être stérilisé sur une cartouche de 0.2$\mu$m. Le filtrat hydroalcoolique est ensuite évaporé sous vide pour éliminer l'éthanol, et la solution aqueuse avec un extrait sec d'environ 10% est atomisée pour obtenir l'extrait de Safran sous la forme d'une poudre fine rouge orangée.

Matériel :

Broyage : Broyeur à couteaux Retsch SM100

Extraction et Alluvionnage : réacteur VSA 40E

Pré-Filtration : filtre clos de 400 mm avec toile Nylon Nylon 195 $\mu$m + recette mobile 50L

Pré-Clarification : filtre agité De Dietrich avec toile coton 10 $\mu$m

Clarification : filtre cloche de 400 mm avec plaque FGC15

Stérilisation : carter avec cartouche filtrante Millipore 0.2 $\mu$m

Concentration : réacteur VSA40E

Atomisation : Büchi mini Spray Dryer B290

Les spécifications de l'extrait ainsi obtenu sont présentés dans le Tableau 1 ci-dessous.

[Tableau 1]

| Ratio bis-gentiobiosyl-E-crocétine/gentiobiosyl-glucosyl-E-crocétine/-bis-glucosyl-E-crocétine/bis-gentiobiosyl-13-Z-crocétine | 62,4/27/6,9/3,9 (détection UV $\lambda$=440 nm) |
|---|---|
| Teneur en crocine totale (exprimée en bis-gentiobiosyl-E-crocétine TC4) | 29,2% |
| Teneur en bis-gentiobiosyl-E-crocétine (TC4) | 18,6% |
| Teneur en gentiobiosyl-glucosyl-E-crocétine TC3 (exprimée en bis-gentiobiosyl-E-crocétine TC4) | 7,9% |
| Teneur en bis-glucosyl-E-crocétine TC2 (exprimée en bis-gentiobiosyl-E-crocétine TC4) | 1,8% |
| Teneur en bis-gentiobiosyl-13-Z-crocétine CC4 (exprimée en bis-gentiobiosyl-E-crocétine TC4) | 0,9% |
| Teneur en safranal | 0% |
| Teneur en picrocrocine | 13,5% |
| Teneur en polyphénols totaux (exprimés en équivalent acide gallique) | 5,2% |
| Teneur en eau | 4,7% |
| TC4 : trans-crocine-4.<br>TC3 : trans-crocine-3.<br>TC2 : trans-crocine-2.<br>CC4 : cis-crocine-4. | |

Teneur en crocine totale (HPLC/DAD, $\lambda$=440nm)

La quantification de la crocine totale et des différentes crocines (TC4 + TC3 + TC2 + CC4) a été réalisée par étalonnage externe par rapport à la trans-crocine-4.

Les résultats sont donc exprimés en trans-crocine-4. Etalon de trans-crocine-4 : Chengdu Biopurify Phytochemicals Ltd, ref. BP0406 lot PRF10071003, pureté 98%. La linéarité de la trans-crocine-4 a été vérifiée entre 0.5 et 100$\mu$g/mL.

La teneur en crocine totale dans l'extrait ainsi obtenu est de 29.2% (exprimé en trans-crocine-4).

Teneur en picrocrocine (HPLC-CAD contre-gradient)

La teneur en picrocrocine a été réalisée par étalonnage externe à partir de l'étalon de trans-crocine-4 utilisé pour quantifier la teneur en crocines.

La teneur en picrocrocine dans l'extrait ainsi obtenu est de 13.5%.

Teneur en safranal (HPLC/DAD, $\lambda$=314nm)

La teneur en safranal dans l'extrait ainsi obtenu est de 0% (non détecté)

Teneur en polyphénols totaux

La teneur en polyphénols totaux a été réalisée par la méthode de Folin en spectrophotométrie. Elle est de 5.2g/100g exprimée en équivalent acide gallique.

**Exemple 2 - Préparation d'un extrait de pistils de *Crocus sativus* provenant de la vallée de l'Ourika comprenant 22,7% de crocines et 0,06% de safranal (selon l'invention).**

Le Safran sous forme de pistils entiers séchés de fleurs de *Crocus Sativus* provenant de la vallée de l'Ourika, récolté en 2018, est broyé avec un broyeur à couteaux sur une grille de 2 mm. 93g de la poudre orangée ainsi obtenue est extraite sous agitation à l'abri de la lumière avec 0,930 litre d'un mélange éthanol-eau (1/1) à 21°C pendant 4h. Le mélange est préfiltré sur une toile Nylon de 50$\mu$m sur un entonnoir Buchner en porcelaine. La biomasse épuisée est dépotée, ré-extraite et pré-filtrée selon le même protocole. Les pré-filtrats issus des deux macérations sont rassemblés, clarifiés sur entonnoir Buchner en porcelaine équipé d'une plaque en cellulose EATON BECO KD3 2,5μm. Le filtrat hydroalcoolique est ensuite évaporé sous vide pour éliminer les solvants puis lyophilisé pour obtenir l'extrait de Safran sous la forme d'une poudre fine rouge vif.

Matériel :

Broyage : Broyeur à couteaux IKA® A11 basic

Extraction : réacteur RADLEY 2,5L

Pré-Filtration : entonnoir Buchner en porcelaine avec toile Nylon Nylon 50 μm

Clarification : entonnoir Buchner en porcelaine avec plaque EATON BECO KD3 2,5μm

Concentration : évaporateur rotatif Buchi Rotavapor R215 équipé d'un bain Buchi HeatingBath B-491, ainsi que d'une pompe à vide Vacuubrand PC 3001 VARIO Pro

Lyophilisation : lyophilisateur Labconco FreeZone 4.5 Plus, couplé à une pompe à vide Vaccubrand RZ-6

Les spécifications de l'extrait ainsi obtenu sont présentés dans le Tableau 2 ci-dessous.

[Tableau 2]

| | |
|---|---|
| Ratio bis-gentiobiosyl-E-crocétine/gentiobiosyl-glucosyl-E-crocétine/bis-glucosyl-E-crocétine/bis-gentiobiosyl- 13-Z-crocétine | 61/27/7,2/4,8 (détection UV λ=440 nm) |
| Teneur en crocine totale (exprimée en bis-gentiobiosyl-E-crocétine TC4) | 22,7% |
| Teneur en bis-gentiobiosyl-E-crocétine (TC4) | 14,1% |
| Teneur en gentiobiosyl-glucosyl-E-crocétine TC3 (exprimée en bis-gentiobiosyl-E-crocétine TC4) | 6,1% |
| Teneur en bis-glucosyl-E-crocétine TC2 (exprimée en bis-gentiobiosyl-E-crocétine TC4) | 1,5% |
| Teneur en bis-gentiobiosyl-13-Z-crocétine CC4 (exprimée en bis-gentiobiosyl-E-crocétine TC4) | 1,0% |
| Teneur en safranal | 0,06% |
| Teneur en picrocrocine | 13,5% |
| Teneur en polyphénols totaux (exprimés en équivalent acide gallique) | 4,6% |
| TC4 : trans-crocine-4.<br>TC3 : trans-crocine-3.<br>TC2 : trans-crocine-2.<br>CC4 : cis-crocine-4. | |

Teneur en crocine totale (HPLC/DAD, λ=440nm)

La quantification de la crocine totale et des différentes crocines (TC4 + TC3 + TC2 + CC4) a été réalisée par étalonnage externe par rapport à la trans-crocine-4.
Les résultats sont donc exprimés en trans-crocine-4. Etalon de trans-crocine-4 : Chengdu Biopurify Phytochemicals Ltd, ref. BP0406, pureté 98%. La linéarité de la trans-crocine-4 a été vérifiée entre 0.5 et 100μg/mL.
La teneur en crocine totale dans l'extrait ainsi obtenu est de 22,7% (exprimé en trans-crocine-4).

Teneur en picrocrocine (HPLC-CAD contre-gradient)

La teneur en picrocrocine a été réalisée par étalonnage externe à partir de l'étalon de trans-crocine-4 utilisé pour quantifier la teneur en crocines.

La teneur en picrocrocine dans l'extrait ainsi obtenu est de 13.5%.

Teneur en safranal (HPLC/DAD, $\lambda$=314nm)

La teneur en safranal dans l'extrait ainsi obtenu est de 0,06%.

Teneur en polyphénols totaux

La teneur en polyphénols totaux a été réalisée par la méthode de Folin en spectrophotométrie. Elle est de 4,6g/100g exprimée en équivalent acide gallique.

**Exemple 3 - Préparation d'un extrait de pistils de *Crocus sativus* provenant de la région de Taliouine comprenant 12% de crocines (hors invention)**

Le Safran sous forme de pistils entiers séchés de fleurs de *Crocus Sativus* provenant de la région de Taliouine, fournisseur Trésors et Saveurs, est broyé avec un broyeur à couteaux sur une grille de 1 mm. 1,5kg de la poudre orangée ainsi obtenue est extraite sous agitation à l'abri de la lumière avec 15 litres d'un mélange éthanol-eau (1/1) à 25°C pendant 4h. Le mélange est préfiltré sur une toile de 50 $\mu$m dans un filtre clos. La biomasse épuisée est dépotée, ré-extraite et préfiltrée selon le même protocole. Les pré-filtrats issus des deux macérations sont rassemblés, pré-clarifiés en présence de cellulose (Vitacel LC200) dans un filtre clos équipé d'une plaque en cellulose de 8-3.5 $\mu$m puis clarifiés dans un filtre cloche équipé d'une plaque en cellulose de 0.8-0.5 $\mu$m et enfin stérilisés à l'aide d'une cartouche de filtration 0.2$\mu$m.

Le filtrat hydroalcoolique stérilisé est ensuite évaporé sous vide pour éliminer l'éthanol et la solution aqueuse légèrement opaque est atomisée pour obtenir l'extrait de Safran sous la forme d'une poudre fine orangée.

Matériel :

Broyage : Broyeur à couteaux Retsch SM100

Extraction : réacteur VSA 20E

Pré-Filtration : filtre clos de 400 mm avec toile Nylon Nitex 50$\mu$m

Pré-Clarification : filtre clos de 400 mm avec plaque FGC7

Clarification : filtre cloche de 400 mm avec plaque FGC15

Stérilisation : cartouche filtrante 0.2$\mu$m

Concentration : réacteur VSA20E

Atomisation : Büchi mini Spray Dryer B290

Les spécifications de l'extrait ainsi obtenu sont présentés dans le Tableau 3 ci-dessous.

[Tableau 3]

| | |
|---|---|
| Ratio bis-gentiobiosyl-E-crocétine/gentiobiosyl-glucosyl-E-crocétine/bis-glucosyl-E-crocétine/bis-gentiobiosyl-13-Z-crocétine | 59/26,5/9/5,5 (détection UV $\lambda$=440 nm) |
| Teneur en crocine totale (exprimée en bis-gentiobiosyl-E-crocétine TC4) | 12,2% |
| Teneur en safranal | 0% |
| Teneur en picrocrocine | 8,1% |
| Teneur en polyphénols totaux (exprimés en équivalent acide gallique) | 4,1% |

(suite)

| Teneur en eau | 5,7% |
|---|---|
| TC4 : trans-crocine-4. | |

Teneur en crocine totale (HPLC/DAD, λ=440nm)

Les étalons des différentes crocines n'étant pas disponibles, la quantification de la crocine totale et des différentes crocines (TC4 + TC3 + TC2 + CC4) a été réalisée par étalonnage externe par rapport à la trans-crocine-4.

Les résultats sont donc exprimés en trans-crocine-4. Etalon de trans-crocine-4 : Chengdu Biopurify Phytochemicals Ltd, ref. BP0406, pureté 98%. La linéarité de la trans-crocine-4 a été vérifiée entre 0.5 et 100μg/mL.

La teneur en crocine totale dans l'extrait ainsi obtenu est de 12.2% (exprimé en trans-crocine-4).

Teneur en picrocrocine (HPLC-CAD contre-gradient)

La teneur en picrocrocine a été réalisée par étalonnage externe à partir d'un étalon de picrocrocine Santa Cruz référence SC-47934/SB lot E0317.

La pureté de l'étalon a été estimée à 74% d'après le profil HPLC-CAD.

La linéarité de la picrocrocine a été vérifiée entre 5 et 200μg/mL.

La teneur en picrocrocine dans l'extrait ainsi obtenu est de 8.1%. La teneur en picrocrocine a été réalisée par étalonnage externe à partir de l'étalon de trans-crocine-4 utilisé pour quantifier la teneur en crocines.

Teneur en safranal (HPLC/DAD, λ=314nm)

La teneur en safranal dans l'extrait ainsi obtenu est de 0% (non détecté)

Teneur en polyphénols totaux

La teneur en polyphénols totaux a été réalisée par la méthode de Folin en spectrophotométrie. Elle est de 4,1g/100g exprimée en équivalent acide gallique.

**Exemple 4 - Evaluation de l'activité antioxydante de l'extrait obtenu selon l'exemple 2 (selon l'invention) par rapport à l'extrait obtenu selon l'exemple 3 (hors invention)**

Etude de la peroxydation du squalène

Protocole : Le principe du test est de déterminer via le dosage du squalène (Sq) et de son produit de photo-oxydation l'efficacité d'un actif contre cette forme de photo-peroxydation.

Le test met à profit du squalène en présence d'un photo-sensibilisant, l'hématoporphyrine. Sous l'action des UVA, de l'oxygène singulet ($^1O_2$) est généré. Cette forme très réactive de l'oxygène dégrade le squalène par oxydation des doubles liaisons. pour former les hydroperoxydes de squalène (Sq-OOH)

Dans le cadre du test, l'exposition du squalène associé à l'hématoporphyrine est réalisée en présence de différentes concentrations en extrait obtenu selon l'exemple 2 (selon l'invention) et selon l'exemple 3 (hors invention). Le milieu réactionnel est l'éthanol.

Matériel et réactifs :

- Squalène [Sigma S-3626]
- Hématoporphyrine Free Base (approx. 70 %) [Sigma - ref H-7253]
- HPLC ou UPLC système en mode phase inverse, détection en mode UV (205nm)

Mode opératoire :

- solution ethanol contenant 500µg/ml de Sq et 2µg/ml hematoporphyrine + Extrait à tester
- exposer sous UVa pour la dose de 5J/cm$^2$
- mesurer quantité de Sq et SqOOH avant et après exposition
- tracer %inhibition en fonction de la quantité d'extrait testé. En déduire si besoin IC25, ou IC50 (quantité d'extrait nécessaire pour réduire 25% ou 50% la réaction d'oxydation du squalène.

[Math. 1]

$$\% \text{ inhibition} = \left[ \frac{\left( \dfrac{SQOOH}{SQ_{résiduel}} \text{ essai} - \dfrac{SQOOH}{SQ_{résiduel}} \text{ témoin} \right)}{\dfrac{SQOOH}{SQ_{résiduel}} \text{ témoin}} \right]_{\text{à 5 Joules/cm2}} \times 100$$

Les résultats sont présentés dans le tableau 3 ci-dessous.

[Tableau 4]

| Matières Premières | Concentration dans le milieu réactionnel | Activité (% inhibition) | Commentaires |
|---|---|---|---|
| Extrait obtenu selon l'exemple 2 (selon l'invention) - solution dans éthanol | 10 ppm<br>26 ppm<br>104 ppm | 17%<br>30%<br>66% | IC25 = 19 ppm<br>IC50 = 56 ppm |
| Extrait obtenu selon l'exemple 3 (hors invention) - solution dans éthanol | 240 ppm | 78% | IC50 = 105 ppm |

L'extrait obtenu selon l'exemple 2 (selon l'invention) présente une forte activité, avec détermination d'une IC25 = 19ppm et d'une IC50 = 56ppm. Cet extrait est donc plus efficace que l'extrait obtenu selon l'exemple 3 (hors invention) qui présente une IC50 = 105ppm bien plus élevée.

En outre, l'extrait obtenu selon l'exemple 2 (selon l'invention) est plus efficace que la Vitamine E, qui a une IC50 de l'ordre de 0,025% (= 250ppm).

*IC25 : concentration en matière première nécessaire pour inhiber la peroxydation du squalène à hauteur de 25%.

**IC50 : concentration en matière première nécessaire pour inhiber la peroxydation du squalène à hauteur de 50%.

**Exemple 5 - Evaluation olfactive de l'extrait obtenu selon l'exemple 2 (selon l'invention) par rapport à l'extrait obtenu selon l'exemple 2' (hors invention)**

L'extrait 2 (selon l'invention) et l'extrait 2' enrichi avec du safranal pur pour atteindre 0.10% en safranal (hors invention) ont été évalués olfactivement par un panel de 4 personnes.

Les caractéristiques olfactives de l'extrait 2' enrichi pour atteindre 0.10% en safranal (hors invention) : plus puissant, chaud, ambré, boisé, épicé, note sale, cacao.

Les caractéristiques olfactives de l'extrait 2 (selon l'invention) moins puissant, plus fruité, miellé, pain, note légume vert.

Il est à noter que, en dehors de la Puissance :

- Ambré, boisé font partie des notes de fond, les plus rémanentes/moins volatiles.
- Epicé, fruité font partie des notes de cœur.

-   Légume vert fait partie des notes de têtes, les plus volatiles.

Ainsi, l'extrait 2 selon l'invention présente une odeur moins puissante que l'extrait 2' hors invention.

**Exemple 6 -Crème pour le visage comprenant l'extrait obtenu selon l'exemple 1 (selon l'invention)**

[Tableau 5]

| | |
|---|---|
| Sorbate de potassium en poudre | 0,1% |
| Xanthane : polysaccharides : glucose/mannane/acide glucuronique (40/30/30) | 0,3% |
| Mélange de plantes à lécithine, acides gras et alcools gras | 5% |
| Extrait de safran obtenu selon l'exemple 1 | 0,1% |
| Huile de tournesol bio première pression à froid | 20% |
| Citrate de stéarate de glycéryle | 2% |
| Alcool benzylique (et) acide déhydroacétique (et) eau | 0,8% |
| Parfum | 0,45% |
| Acide citrique | 0,1% |
| Eau (q.s.p.) | 100% |

**Revendications**

1.  Extrait d'au moins une plante de l'espèce *Crocus sativus* comprenant (i) au moins 20% en poids sec d'une ou plusieurs crocine(s) par rapport au poids total des composants de l'extrait sec et (ii) moins de 0,08% en poids sec de safranal par rapport au poids total des composants de l'extrait sec, dans lequel la ou les crocine(s) est(sont) sous la forme d'un mélange comprenant :

    - entre 10% et 30% en poids sec de bis-gentiobiosyl-E-crocétine ;
    - entre 5% et 10% en poids sec de gentiobiosyl-glucosyl-E-crocétine ;
    - entre 0,1% et 5% en poids sec de bis-glucosyl-E-crocétine ;
    - entre 0,05% et 2% en poids sec de bis-gentiobiosyl-13-Z-crocétine ;

    par rapport au poids total des composants de l'extrait sec.

2.  Extrait selon la revendication 1, dans lequel ladite ou lesdites crocine(s) (i) est(sont) présente(s) entre 20% et 40% en poids sec par rapport au poids total des composants de l'extrait sec, de préférence, entre 25% et 35% en poids sec par rapport au poids total des composants de l'extrait sec.

3.  Extrait selon la revendication 1 ou 2, dans lequel ledit safranal (ii) est présent à moins de 0,07% en poids sec par rapport au poids total des composants de l'extrait sec, de préférence, à moins de 0,06% en poids sec par rapport au poids total des composants de l'extrait sec.

4.  Extrait selon l'une quelconque des revendications précédentes, dans lequel la ou les crocine(s) est(sont) sous la forme d'un mélange comprenant :

    - entre 60% et 70% en poids sec de bis-gentiobiosyl-E-crocétine ;
    - entre 20% et 30% en poids sec de gentiobiosyl-glucosyl-E-crocétine ;
    - entre 5% et 10% en poids sec de bis-glucosyl-E-crocétine ;
    - entre 2% et 6% en poids sec de bis-gentiobiosyl-13-Z-crocétine ;

    par rapport au poids total des crocines de l'extrait sec.

**5.** Extrait selon l'une quelconque des revendications précédentes, dans lequel ladite plante de l'espèce *Crocus sativus* provient du Maroc, notamment de la vallée de l'Ourika.

**6.** Composition comprenant, dans un milieu physiologiquement acceptable l'extrait tel que défini selon l'une quelconque des revendications 1 à 5.

**7.** Composition selon la revendication 6, dans laquelle ledit extrait est présent dans la composition en une concentration comprise entre 0,0001% et 20% en poids d'extrait sec, de préférence entre 0,001% et 10% en poids d'extrait sec, encore mieux entre 0,001% et 5% en poids d'extrait sec par rapport au poids total de la composition.

**8.** Procédé de traitement cosmétique non thérapeutique comprenant l'application sur les matières kératiniques, notamment la peau, de l'extrait tel que défini selon l'une quelconque des revendications 1 à 5 ou de la composition telle que définie selon l'une quelconque des revendications 6 ou 7, pour le soin de la peau, et notamment pour prévenir et/ou traiter l'aspect terne et/ou hétérogène du teint, et/ou pour améliorer l'éclat et/ou l'uniformité du teint, et/ou pour prévenir les désordres pigmentaires tels que le lentigo actinique.

**9.** Procédé de traitement cosmétique non- thérapeutique comprenant l'application sur les matières kératiniques, notamment la peau, de l'extrait tel que défini selon l'une quelconque des revendications 1 à 5 ou de la composition telle que définie selon l'une quelconque des revendications 6 ou 7, pour prévenir et/ou traiter les signes du vieillissement cutané.

**10.** Utilisation cosmétique non- thérapeutique de l'extrait tel que défini selon l'une quelconque des revendications 1 à 5 comme agent antioxydant.

**11.** Utilisation cosmétique non thérapeutique de l'extrait tel que défini selon l'une quelconque des revendications 1 à 5 pour le soin de la peau, et notamment pour prévenir et/ou traiter l'aspect terne et/ou hétérogène du teint, et/ou pour améliorer l'éclat et/ou l'uniformité du teint, et/ou pour prévenir les désordres pigmentaires tels que le lentigo actinique.

**12.** Utilisation cosmétique non- thérapeutique de l'extrait tel que défini selon l'une quelconque des revendications 1 à 5 pour prévenir et/ou traiter les signes du vieillissement cutané.

**13.** Procédé de préparation d'un extrait d'au moins une plante de l'espèce *Crocus sativus* tel que défini selon l'une quelconque des revendications 1 à 5 comprenant au moins une étape d'extraction d'une partie d'au moins une plante de l'espèce *Crocus sativus* à l'aide d'un solvant aqueux choisi parmi l'eau ou un mélange d'eau et d'un ou plusieurs solvant(s) organique(s) miscible(s) dans l'eau, de préférence à l'aide d'un mélange éthanol/eau dans un ratio en volume de 1/1.

**14.** Procédé selon la revendication 13, dans lequel l'extrait obtenu à l'issue de ladite étape d'extraction peut subir les étapes suivantes, de préférence consécutives :

    i. une étape de filtration, en particulier sur une toile de nylon présentant une taille de pores comprise entre $50\mu m$ et $200\mu m$, et/ou
    ii. une étape d'empâtage dans de la cellulose, et/ou
    iii. une ou plusieurs étapes de clarification, de préférence l'extrait obtenu à l'issue de l'étape d'extraction peut subir une première étape de clarification sur un lit de cellulose et toile de coton présentant une taille de pores comprise entre $5\mu m$ et $15\mu m$ ainsi qu'une seconde étape de clarification sur une plaque de cellulose présentant une taille de pores comprise entre $0,1\mu m$ et $3\mu m$, de préférence entre $0,8\mu m$ et $0,5\mu m$ ; et/ou
    iv. une étape de stérilisation, notamment par passage à travers une cartouche de $0,2\mu m$.

**15.** Procédé selon la revendication 13 ou 14, dans lequel à l'issue de ladite étape d'extraction et éventuellement des étapes i) et/ou ii) et/ou iii) et/ou iv), de préférence consécutives, ledit extrait subi une étape de séchage qui comprend l'élimination d'un(des) solvant(s), par exemple par évaporation d'un ou plusieurs solvants organiques miscibles dans l'eau notamment à l'aide d'un évaporateur rotatif et/ou par évaporation de l'eau notamment à l'aide d'un évaporateur rotatif, par lyophilisation, ou par atomisation, ou une combinaison de ces méthodes.

**Patentansprüche**

1. Extrakt aus mindestens einer Pflanze der Art Crocus sativus, umfassend (i) mindestens 20 % nach Trockengewicht an einem oder mehreren Crocin(en), bezogen auf das Gesamtgewicht der Bestandteile des Trockenextrakts, und (ii) weniger als 0,08 % nach Trockengewicht an Safranal, bezogen auf das Gesamtgewicht der Bestandteile des Trockenextrakts, wobei das/die Crocin(e) in Form einer Mischung vorliegt/vorliegen, die Folgendes umfasst:

   - 10 % bis 30 % nach Trockengewicht an bis-Gentiobiosyl-E-crocetin;
   - 5 % bis 10 % nach Trockengewicht an Gentiobiosyl-glucosyl-E-crocetin;
   - 0,1 % bis 5 % nach Trockengewicht an bis-Glucosyl-E-crocetin;
   - 0,05 % bis 2 % nach Trockengewicht an bis-Gentiobiosyl-13-Z-crocetin;

   bezogen auf das Gesamtgewicht der Bestandteile des Trockenextrakts.

2. Extrakt nach Anspruch 1, wobei das/die Crocin(e) (i) 20 % bis 40 % nach Trockengewicht, bezogen auf das Gesamtgewicht der Bestandteile des Trockenextrakts, vorzugsweise 25 % bis 35 % nach Trockengewicht, bezogen auf das Gesamtgewicht der Bestandteile des Trockenextrakts, ausmacht/ausmachen.

3. Extrakt nach Anspruch 1 oder 2, wobei das Safranal (ii) weniger als 0,07 % nach Trockengewicht, bezogen auf das Gesamtgewicht der Bestandteile des Trockenextrakts, vorzugsweise weniger als 0,06 % nach Trockengewicht, bezogen auf das Gesamtgewicht der Bestandteile des Trockenextrakts, ausmacht.

4. Extrakt nach einem beliebigen der vorhergehenden Ansprüche, wobei das/die Crocin(e) in Form einer Mischung vorliegt/vorliegen, die Folgendes umfasst:

   - 60 % bis 70 % nach Trockengewicht an bis-Gentiobiosyl-E-crocetin;
   - 20 % bis 30 % nach Trockengewicht an Gentiobiosyl-glucosyl-E-crocetin;
   - 5 % bis 10 % nach Trockengewicht an bis-Glucosyl-E-crocetin;
   - 2 % bis 6 % nach Trockengewicht an bis-Gentiobiosyl-13-Z-crocetin;

   bezogen auf das Gesamtgewicht der Crocine des Trockenextrakts.

5. Extrakt nach einem beliebigen der vorhergehenden Ansprüche, wobei die Pflanze der Art Crocus sativus aus Marokko stammt, insbesondere aus dem Ourika-Tal.

6. Zusammensetzung, die den Extrakt gemäß der Begriffsbestimmung in einem der Ansprüche 1 bis 5 in einem physiologisch unbedenklichen Medium umfasst.

7. Zusammensetzung nach Anspruch 6, wobei der Extrakt in der Zusammensetzung in einer Konzentration im Bereich von 0,0001 % bis 20 % nach Gewicht an Trockenextrakt, vorzugsweise von 0,001 % bis 10 % nach Gewicht an Trockenextrakt, besser noch von 0,001 % bis 5 % nach Gewicht an Trockenextrakt vorliegt, bezogen auf das Gesamtgewicht der Zusammensetzung.

8. Nicht-therapeutisches kosmetisches Behandlungsverfahren, das ein Aufbringen des Extrakts gemäß der Begriffsbestimmung in einem beliebigen der Ansprüche 1 bis 5 oder der Zusammensetzung gemäß der Begriffsbestimmung in einem beliebigen der Ansprüche 6 oder 7 auf Keratinmaterialien, insbesondere die Haut, umfasst, um die Haut zu pflegen und insbesondere ein stumpfes und/oder ungleichmäßiges Aussehen des Hautbildes zu verhüten und/oder zu behandeln und/oder die Strahlkraft und/oder Gleichmäßigkeit des Hautbildes zu verbessern und/oder Pigmentstörungen wie Lentigo solaris zu verhüten.

9. Nicht-therapeutisches kosmetisches Behandlungsverfahren, das ein Aufbringen des Extrakts gemäß der Begriffsbestimmung in einem beliebigen der Ansprüche 1 bis 5 oder der Zusammensetzung gemäß der Begriffsbestimmung in einem beliebigen der Ansprüche 6 oder 7 auf Keratinmaterialien, insbesondere die Haut, umfasst, um Anzeichen der Hautalterung zu verhüten und/oder zu behandeln.

10. Nicht-therapeutische kosmetische Verwendung des Extrakts gemäß der Begriffsbestimmung in einem beliebigen der Ansprüche 1 bis 5 als Antioxidans.

11. Nicht-therapeutische kosmetische Verwendung des Extrakts gemäß der Begriffsbestimmung in einem beliebigen der Ansprüche 1 bis 5 zur Pflege der Haut, und insbesondere um ein stumpfes und/oder ungleichmäßiges Aussehen des Hautbildes zu verhüten und/oder zu behandeln und/oder die Strahlkraft und/oder Gleichmäßigkeit des Hautbildes zu verbessern und/oder Pigmentstörungen wie Lentigo solaris zu verhüten.

12. Nicht-therapeutische kosmetische Verwendung des Extrakts gemäß der Begriffsbestimmung in einem beliebigen der Ansprüche 1 bis 5, um Anzeichen der Hautalterung zu verhüten und/oder zu behandeln.

13. Verfahren zur Herstellung eines Extrakts mindestens einer Pflanze der Art Crocus sativus gemäß der Begriffsbestimmung in einem beliebigen der Ansprüche 1 bis 5, wobei es mindestens einen Schritt des Extrahierens eines Teils zumindest einer Pflanze der Art Crocus sativus unter Verwendung eines wässrigen Lösungsmittels, welches aus Wasser oder einer Mischung aus Wasser und einem oder mehreren mit Wasser mischbaren organischen Lösungsmittel(n) ausgewählt ist, vorzugsweise unter Verwendung einer Ethanol/Wasser-Mischung im Volumenverhältnis 1/1 umfasst.

14. Verfahren nach Anspruch 13, wobei der Extrakt, welcher nach Abschluss des Extraktionsschritt erhalten wurde, den folgenden, vorzugsweise aufeinanderfolgenden Schritten unterzogen werden kann:

    i. einem Filtrationsschritt, insbesondere über ein Nylongewebe mit einer Porengröße im Bereich von 50 $\mu$m bis 200 $\mu$m, und/oder
    ii. einem Schritt des Anrührens in Cellulose und/oder
    iii. einem oder mehreren Klärungsschritten, wobei der Extrakt, welcher nach Abschluss des Extraktionsschrittes erhalten wurde, vorzugsweise einem ersten Klärungsschritt über ein Bett aus Cellulose und Baumwolltuch mit einer Porengröße im Bereich von 5 $\mu$m bis 15 $\mu$m sowie einem zweiten Klärungsschritt über eine Celluloseplatte mit einer Porengröße im Bereich von 0,1 $\mu$m bis 3 $\mu$m, vorzugsweise im Bereich von 0,8 $\mu$m bis 0,5 $\mu$m, unterzogen werden kann; und/oder
    iv. einem Sterilisationsschritt, insbesondere indem er durch eine 0,2-$\mu$m-Kartusche geleitet wird.

15. Verfahren nach Anspruch 13 oder 14, wobei nach Abschluss des Extraktionsschritts und gegebenenfalls der Schritte i) und/oder ii) und/oder iii) und/oder iv), welche vorzugsweise nacheinander erfolgen, der Extrakt einem Trocknungsschritt unterzogen wird, welcher das Entfernen eines Lösungsmittels/der Lösungsmittel umfasst, indem beispielsweise ein oder mehrere mit Wasser mischbare organische Lösungsmittel insbesondere mit Hilfe eines Rotationsverdampfers verdampft werden und/oder indem Wasser insbesondere mit Hilfe eines Rotationsverdampfers verdampft wird, durch Gefriertrocknung oder durch Zerstäubung oder eine Kombination dieser Methoden.

**Claims**

1. Extract of at least one plant of the *Crocus sativus* species comprising (i) at least 20% by dry weight of one of more crocin(s) relative to the total weight of the components of the dry extract and (ii) less than 0.08% by dry weight of safranal relative to the total weight of the components of the dry extract, in which the crocin(s) is(are) in the form of a mixture comprising:

    - between 10% and 30% by dry weight of bis-gentiobiosyl-E-crocetin;
    - between 5% and 10% by dry weight of gentiobiosyl-glucosyl-E-crocetin;
    - between 0.1% and 5% by dry weight of bis-glucosyl-E-crocetin;
    - between 0.05% and 2% by dry weight of bis-gentiobiosyl-13-Z-crocetin;

    relative to the total weight of the components of the dry extract.

2. Extract according to Claim 1, in which said crocin(s) (i) is(are) present at between 20% and 40% by dry weight relative to the total weight of the components of the dry extract, preferably between 25% and 35% by dry weight relative to the total weight of the components of the dry extract.

3. Extract according to Claim 1 or 2, in which said safranal (ii) is present at less than 0.07% by dry weight relative to the total weight of the components of the dry extract, preferably at less than 0.06% by dry weight relative to the total weight of the components of the dry extract.

4. Process according to any one of the preceding claims, in which the

    crocin(s) is(are) in the form of a mixture comprising:

    - between 60% and 70% by dry weight of bis-gentiobiosyl-E-crocetin;
    - between 20% and 30% by dry weight of gentiobiosyl-glucosyl-E-crocetin;
    - between 5% and 10% by dry weight of bis-glucosyl-E-crocetin;
    - between 2% and 6% by dry weight of bis-gentiobiosyl-13-Z-crocetin;

    relative to the total weight of the crocins in the dry extract.

5. Extract according to any one of the preceding claims, in which said plant of the *Crocus sativus* species originates from Morocco, notably from the Ourika Valley.

6. Composition comprising, in a physiologically acceptable medium, the extract as defined according to any one of Claims 1 to 5.

7. Composition according to Claim 6, in which said extract is present in the composition in a concentration of between 0.0001% and 20% by weight of dry extract, preferably between 0.001% and 10% by weight of dry extract and even better still between 0.001% and 5% by weight of dry extract relative to the total weight of the composition.

8. Non-therapeutic cosmetic treatment process comprising the application to keratin materials, notably the skin, of the extract as defined according to any one of Claims 1 to 5 or of the composition as defined according to either one of Claims 6 and 7, for skincare, and notably to prevent and/or treat the dull and/or heterogeneous appearance of the complexion, and/or to improve the radiance and/or uniformity of the complexion, and/or to prevent pigment disorders such as actinic lentigo.

9. Non-therapeutic cosmetic treatment process comprising the application to keratin materials, notably the skin, of the extract as defined according to any one of Claims 1 to 5 or of the composition as defined according to either one of Claims 6 and 7, to prevent and/or treat the signs of skin ageing.

10. Non-therapeutic cosmetic use of the extract as defined according to any one of Claims 1 to 5, as an antioxidant.

11. Non-therapeutic cosmetic use of the extract as defined according to any one of Claims 1 to 5 for skincare, and notably to prevent and/or treat the dull and/or heterogeneous appearance of the complexion, and/or to improve the radiance and/or uniformity of the complexion, and/or to prevent pigment disorders such as actinic lentigo.

12. Non-therapeutic cosmetic use of the extract as defined according to any one of Claims 1 to 5 to prevent and/or treat the signs of skin ageing.

13. Process for preparing an extract of at least one plant of the *Crocus sativus* species as defined according to any one of Claims 1 to 5 comprising at least one step of extracting a part of at least one plant of the *Crocus sativus* species using an aqueous solvent chosen from water or a mixture of water and one or more water-miscible organic solvent(s), preferably using an ethanol/water mixture in a volume ratio of 1/1.

14. Process according to Claim 13, in which the extract obtained on conclusion of said extraction step may be subjected to the following, preferably consecutive, steps:

    i. a step of filtration, in particular on a nylon gauze having a pore size of between 50 $\mu$m and 200 $\mu$m, and/or
    ii. a step of slurrying in cellulose, and/or
    iii. one or more clarification steps; preferably, the extract obtained on conclusion of the extraction step may be subjected to a first step of clarification on a bed of cellulose and cotton gauze having a pore size of between 5 $\mu$m and 15 $\mu$m and also a second step of clarification on a cellulose sheet having a pore size of between 0.1 $\mu$m and 3 $\mu$m, preferably between 0.8 $\mu$m and 0.5 $\mu$m; and/or
    iv. a step of sterilization, notably by passage through a 0.2 $\mu$m cartridge.

15. Process according to Claim 13 or 14, in which, on conclusion of said extraction step and optionally of steps i) and/or ii) and/or iii) and/or iv), which are preferably consecutive, said extract is subjected to a drying step which comprises the removal of one(of the) solvent(s), for example by evaporation of one or more water-miscible organic solvents, notably using a rotary evaporator and/or by evaporation of the water notably using a rotary evaporator, by freeze drying, or by spray drying, or a combination of these methods.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3001891 A1
- FR 2949975 A1
- JP 2005041811 A
- JP 2005343882 A
- CN 108542840 A

**Littérature non-brevet citée dans la description**

- **KEIKO OH SAWA et al.** *J. Toxicol. Sc.*, 1984, vol. 19, 151-159
- **MATTHIAS LECHTENBERG** ; **DIRK SCHEPMANN** ; **MICHAEL NIEHUES** ; **NILS HELLENBRAND** ; **BERNHARD WÜNSCH** ; **ANDREAS HENSEL**. *journal Planta Medica*, June 2008